(19) Europäisches Patentamt
European Patent Office
Office européen des brevets

(11) Veröffentlichungsnummer: **0 605 810 A1**

# (12) EUROPÄISCHE PATENTANMELDUNG

(21) Anmeldenummer: **93119935.0**

(51) Int. Cl.[5]: **C07C 29/151**

(22) Anmeldetag: **10.12.93**

(30) Priorität: **02.01.93 DE 4300017**

(43) Veröffentlichungstag der Anmeldung:
**13.07.94 Patentblatt 94/28**

(84) Benannte Vertragsstaaten:
**AT BE CH DE DK ES FR GB GR IT LI NL SE**

(71) Anmelder: **RWE Entsorgung Aktiengesellschaft**
**Bamlerstrasse 61**
**D-45141 Essen(DE)**

(72) Erfinder: **Münch, Herbert, Dr.**
**Waterloostrasse 39**
**D-45141 Essen(DE)**
Erfinder: **Herbermann, Michael, Dr.**
**Frielinghausstrasse 43**
**D-45966 Gladbeck(DE)**
Erfinder: **Wintrich, Franz, Dipl.-Ing.**
**Berkenberg 25 a**
**D-45309 Essen(DE)**

## (54) Verfahren zur Herstellung von Methanol aus Abfällen.

(57) Die vorliegende Erfindung betrifft ein Verfahren zur Herstellung von Methanol durch Vergasen Kohlenstoff enthaltender Abfälle, Reinigung des auf diese Weise erzeugten Synthesegases und Einsatz des Synthesegases in ein Reaktorsystem mit Zwischenabzug von erzeugtem Methanol.

Figur

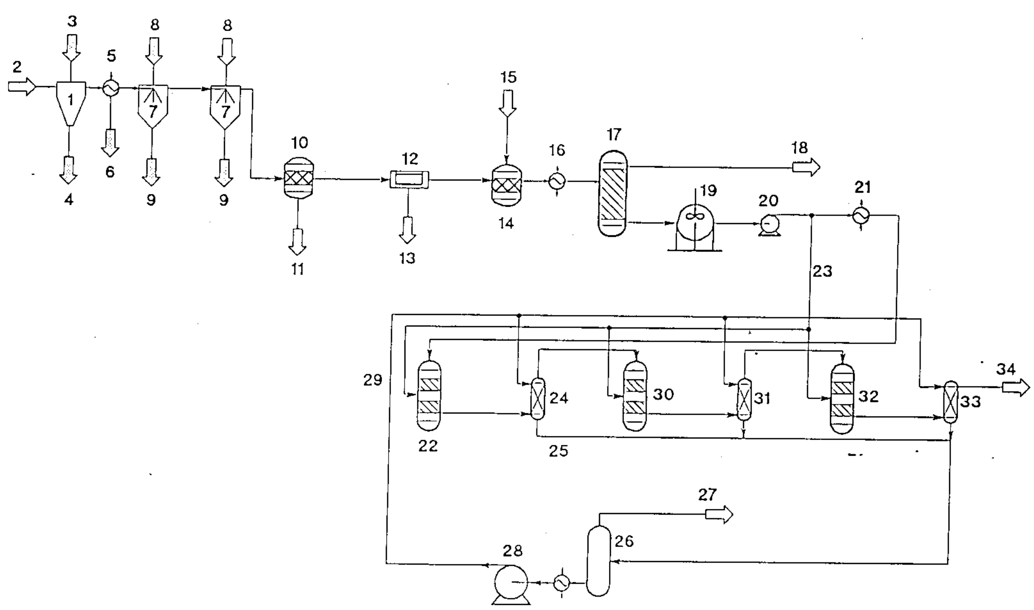




Rank Xerox (UK) Business Services
(3.10/3.09/3.3.4)

Die vorliegende Erfindung betrifft ein Verfahren zur Herstellung von Methanol durch Vergasen Kohlenstoff enthaltender Abfälle, Reststoffe und aus Abfällen und Reststoffen stammender Sekundärrohstoffe, Reinigung des auf diese Weise erzeugten Synthesegases und Einsatz des Synthesegases in ein Reaktorsystem mit Zwischenabzug von erzeugtem Methanol.

Obgleich auf die Verbrennung von Abfällen, insbesondere von Sondermüll, bisher nicht verzichtet werden kann, bemüht sich die Fachwelt intensiv um andere großtechnische Entsorgungstechnologien für Kohlenstoff enthaltende Abfälle, wobei die Gewinnung wertvoller Rohstoffe durch diese Technologien im Vordergrund steht.

Zunehmende Bedeutung erlangt in diesem Zusammenhang die Vergasung Kohlenstoff enthaltender Abfälle, d. h. die endotherme Umsetzung der Abfälle mit Wasserdampf, wobei die erforderliche hohe Temperatur durch Umsetzung eines Teils des Abfalls mit zudosiertem Sauerstoff erfolgt.

Vergasungsreaktoren für schwere Rückstandsöle und auch für schlammartig angemaischte, fein verteilte Kohle, sind seit langem Stand der Technik, wobei die Vergasung von Rückstandsölen, die weitaus wichtigste Technologie zur Erzeugung von Synthesegas, ein Gemisch von Kohlenmonoxyd und Wasserstoff, ist.

Synthesegas ist ein sehr wichtiger Rohstoff für die Erzeugung von Chemikalien, wie Methanol, Oxoalkoholen, Essigsäureanhydrid und insbesondere von Wasserstoff und damit auch für die Erzeugung von Ammoniak und Harnstoff.

Um Kohlenstoff enthaltende Abfälle in üblichen Vergasern zu vergasen, ist die Zerkleinerung derselben eine Voraussetzung, einmal um sie schlammartig oder als Pulver in den Vergaser eintragen zu können und zum anderen, um eine möglichst vollständige Umsetzung zu erzielen. Die Zerkleinerung ist jedoch mit hohem technischem und damit auch wirtschaftlichem Aufwand verbunden.

Umfangreiche Abfallvergasungsversuche wurden von Texaco Inc. durchgeführt. Die zerkleinerten Abfälle wurden im Gemisch mit zerkleinerter Kohle als Slurry (Schlamm) in den Vergaser eingeführt. Beispielhaft sei auf eine Veröffentlichung hingewiesen: L.A. Davis, J.E. Miranda, Monte Bello Research Laboratory, Texaco Inc., Montebello, California, 90640, März 1960, "Texaco hazardous waste gasification".

Auch die Vergasung von getrocknetem Klärschlamm durch Flugstromvergasung ist bekannt. Hierbei wird gemahlener, trockener Klärschlamm mit einem Trägergas in den Vergasungsreaktor eingeführt.

Die Erzeugung von Bulk-Chemikalien, insbesondere von Methanol aus Abfällen, verteuert sich durch die aufwendige Zerkleinerung, aber auch durch das Einbringen großer Mengen Trägergas in den Vergasungsreaktor sehr erheblich. Hierbei ist zu berücksichtigen, daß auch in den heute betriebenen Niederdruckmethanolsynthesen, wie z. B. dem LURGI-Verfahren (ca. 60 bar) oder dem ICI-Verfahren (ca. 100 bar) durch die Kreislaufgas-Fahrweise zusätzliche hohe Kosten entstehen.

Es bestand daher für den Fachmann die Aufgabe, die Wirtschaftlichkeit der Methanolsynthese, ausgehend von Kohlenstoff enthaltenden Abfällen und verwandten Materialien, weiter zu verbessern. Dies ist insbesondere von sehr großer Bedeutung, wenn Methanol als Kraftstoff alternativ zu Benzin und Dieselöl eingesetzt werden soll. Da Methanol zu 50 Gew.-% aus Sauerstoff besteht, müssen die Erzeugungskosten des Methanols, trotz höherem Wirkungsgrad als Kraftstoff, erheblich unter den Erzeugungskosten von Benzin und Dieselöl liegen (siehe z. B. "Alternative Energien für den Straßenverkehr, Methanol", Herausgeber: Der Bundesminister für Forschung und Technologie, Verlag: TÜV-Rheinland GmbH, Köln, 1983).

Der Anmelderin ist es durch die vorliegende Erfindung gelungen, eine entscheidende Verbesserung herbeizuführen, die es ermöglicht, aus Kohlenstoff enthaltenden Abfällen Methanol, das z. B. als besonders emissionsarmer alternativer Kraftstoff genutzt werden kann, wesentlich günstiger herzustellen als dies bisher möglich war, nämlich durch ein Verfahren zur Herstellung von Methanol durch Vergasen Kohlenstoff enthaltender Materialien, Reinigung des erzeugten Synthesegases und Umsetzung des Synthesegases zu Methanol, dadurch gekennzeichnet, daß als Kohlenstoff enthaltende Materialien, Kohlenstoff enthaltende Abfälle und/oder Reststoffe und/oder aus Abfällen und Reststoffen stammende Sekundärrohstoffe vergast werden und daß die Umsetzung des auf diese Weise erzeugten Synthesegases zu Methanol in einem Reaktorsystem mit Zwischenabzug von Methanol erfolgt.

Die vorliegende Erfindung besteht demgemäß aus der Kombination der Vergasung von Abfällen in einer Vergasungsanlage, die ein Eintragsystem besitzt, welches es erlaubt, ohne Feinzerkleinerung des Abfalls, dieses Einsatzmaterial dem Vergasungsreaktor zuzuführen, mit einem Verfahren zur Methanolerzeugung, bei dem Kompressionskosten weitgehend entfallen. Zusätzlich ist ein Gasreinigungsverfahren erfindungsgemäß bevorzugt, das wesentlich geringere Invest- und Betriebskosten erfordert als übliche Gaswäschen, wie z. B. das Rectisol-Verfahren.

"Feinzerkleinerung" bezieht sich auf die Korngröße des Einsatzmaterials in den Vergasungsreaktor selbst. Die Korngröße darf z. B. bei Slurryfahrweise und Flugstromvergasung 0,5 mm nicht überschreiten. Bei der Wirbelschichtvergasung von

Kohle werden zwar Korngrößen von bis 6 mm eingesetzt. Diese Korngröße ist jedoch erforderlich, um das Wirbelbett aufrechtzuerhalten. Um eine vollständige Vergasung zu erzielen, muß jedoch ein Teil des im Zyclon hinter dem Vergaser abgeschiedenen Staubs in den Reaktor rückgeführt werden.

Erfindungsgemäß bevorzugte Vergasungsverfahren sind das Thermoselect-Verfahren bzw. Verfahren, die nach gleichem oder ähnlichem Prinzip arbeiten.

Das Thermoselect-Verfahren enthält einen beheizten Entgasungskanal, in dem verdichtete Abfälle unter Luftausschluß entgast werden. Gleichzeitig tritt zunehmend Verkohlung ein. Die Abfälle können beliebige Gemische sein und auch anorganische Bestandteile enthalten. Eine Zerkleinerung ist nicht erforderlich. So können beispielsweise Autoreifen nur grob zerkleinert, stückig zugeführt werden, ferner Hausmüll einschließlich der vegetabilischen, Plastik-, Chlor- und andere Komponenten enthaltenden Anteile, ohne daß eine aufwendige Sortierung erforderlich ist.

Die entgasten organischen und anorganischen Materialien werden anschließend bei Temperaturen von etwa 1400 - 2000 °C, wobei in der heißesten Zone die Temperatur auch oberhalb 2000 °C liegen kann, in einem Hochtemperaturreaktor unter dosierter Sauerstoffzugabe vergast bzw. verflüssigt.

Auch das im Entgasungskanal entstehende Gas wird dem Hochtemperaturreaktor zugeführt. Die flüssige Schlacke wird einem Homogenisierungsreaktor zugeführt, der bei vergleichbarer Temperatur wie der Vergasungsreaktor arbeitet, in dem aufgrund der unterschiedlichen Dichte eine Trennung in flüssiges Metall und in flüssige Schlacke eintritt. Die Schlacke läßt man zu festem, glasartigem, nicht eluierbarem Material erstarren, der zweite Reaktor wird bevorzugt mit Synthesegas und Sauerstoff beheizt. Das entstehende $CO_2$ gelangt bevorzugt in den ersten Hochtemperaturreaktor.

Das Thermoselect-Verfahren ist beispielsweise in der Patentschrift P 4130416 näher beschrieben.

Ein weiterer, erfindungsgemäß bevorzugter Vergasungsreaktor ist ein Zyklonreaktor, in dein die Abfallmaterialien, Sauerstoff und Dampf tangential in den Vergasungsreaktor eingeführt werden. Da in dem Reaktor stark turbulente Strömung und hierdurch intensive Durchmischung eintritt, lassen sich Abfallmaterialien bis zu einer Größe von ca. 15 mm vergasen.

Es ist dem Fachmann bekannt, daß auch hier kleinere Korngrößen eingesetzt werden können, der wirtschaftliche Aufwand wird hierdurch jedoch erhöht.

Beispielhaft sei auf den Zyklonvergaser hingewiesen, der in der europäischen Patentanmeldung Nr. 92111106.8 offenbart ist. In diesem Reaktor tritt eine Strömung auf, die nicht nur aus einer tangentialen, einer zum Zyklonausgang wirkenden und einer radialen Komponente besteht, sondern in der Strömung tritt zusätzlich ein Drall unter Wirbelausbildung auf, wobei die Drallstärke zu sogenanntem Wirbelaufplatzen der Strömung führt, d. h. es treten Strömungsablösungen bzw. -instabilitäten auf, die zu hohen Turbulenzen im Reaktor führen, wobei der Drall

$$S = \frac{D}{I \times R}$$

ist.

Hier ist D der Drehimpulsstrom, I der Axialimpulsstrom und R der Kammerradius. In einer solchen Strömung treten für die Vergasung besonders günstige kinetische Verhältnisse auf, so daß eine starke Zerkleinerung des Abfalleinsatzprodukts nicht erforderlich ist.

Als erfindungsgemäß vergasbare Abfälle seien beispielhaft und nicht limitierend genannt: Kunststoffabfälle bzw. Kunststoffsekundärrohstoffe, wie Thermoplaste, Duroplaste, Elastomere, Autoreifen, Shredderabfälle, Textilabfälle, Kabelabfälle, Spuckstoffe, Papier- und Pappeabfälle, Industrieschlämme, Klärschlämme, organische Chemikalien, Krankenhausabfälle, Siedlungsabfälle, Altöle, Gefrierschutzmittel, Schmiermittelabfälle, Lack- und Farbreste, Lebensmittelabfälle, forstwirtschaftliche Abfälle u. a.

Die Abfälle können erfindungsgemäß auch gemeinsam mit beliebigen sonstigen Kohlenstoff enthaltenden Materialien eingesetzt werden, wie z. B. mit Biomasse, Kohlen aller Art, Asphalten, Ölsanden, Ölschiefer, schweren Rückstandsölen, Bitumen, Koks u. a.

Bei der Synthese von Methanol stellt sich bekanntlich an dem Katalysator ein von Druck und Temperatur abhängiges Gleichgewicht ein.

$$CO + 2H_2 \rightleftharpoons CH_3OH + \Delta H$$

Die Methanolbildung ist stark exotherm.

Durch die insbesondere von ICI entwickelte Feinstreinigung von Synthesegas über Zinkoxydbetten lassen sich bei den modernen Methanolsynthese-Verfahren hochaktive, Kupfer enthaltende Katalysatoren einsetzen. Man kann daher bei niedrigeren Temperaturen arbeiten als bei den Hochdrucksynthesen, wobei die niedrigeren Temperaturen die Gleichgewichtseinstellung mit höherer Methanolkonzentration im Gleichgewicht begünstigen.

Hierdurch kann man auf die in den früheren Methanolhochdrucksynthese-Anlagen angewendeten Drücke von ca. 300 bis 350 bar verzichten und bereits bei 60 bis 100 bar Methanol technisch erzeugen. Noch immer ist jedoch die Rückführung einer großen aus CO und $H_2$ bestehenden Kreislaufgasmenge erforderlich, da das aus dem Reaktor austretende Gleichgewichtsgemisch mit Methanol zunächst abgekühlt wird, das Methanol durch Kondensation abgetrennt wird und das nunmehr nicht mehr zur Gleichgewichtseinstellung befähigte Synthesegas, zunächst durch Wärmetausch erneut auf Reaktionstemperatur aufgeheizt werden muß und dann erneut über den Katalysator geleitet werden muß.

Im Falle des LURGI-Verfahrens wird nur ein Volumenteil des Synthesegases zu Methanol im geraden Durchgang durch den Reaktor umgesetzt, während 4 bis 6 Volumenteile im Kreislauf gefahren werden.

Obgleich sich die Kompressionskosten gegenüber der früheren Hochdruckfahrweise deutlich vermindert haben, ist der Kompressionskostenanteil noch immer ein sehr wichtiger Kostenfaktor. Ferner erfordert das Kreislaufsystem hohe Investitionskosten.

Ein wesentlicher Bestandteil der erfindungsgemäßen Kombination von Verfahrensschritten zur Methanolerzeugung von bisher nicht bekannter Wirtschaftlichkeit aus Abfällen und verwandten Materialien als Einsatzmaterialien, ist daher ein Verfahren, bei dem man auf die Kreislauffahrweise verzichten kann, Kompressionskosten, Katalysatorkosten sowie erhebliche Investkosten einspart unter Verzicht auf das Kreislaufsystem und durch Verminderung des Reaktorvolumens.

Ein solches Verfahren ist erfindungsgemäß eine Methanolsynthese, bei der das erzeugte Methanol während der katalytischen Umsetzung des Synthesegases aus dem Gleichgewicht entfernt wird. Dies kann auf unterschiedliche Weise geschehen. Beispielhaft sei die Methanolsynthese in einem Rieselreaktor genannt, in dem ein pulverförmiges Adsorptionsmittel zwischen den Katalysatorpartikeln von oben nach unten durch den Reaktor fließt und hierbei das gebildete Methanol adsorbiert (siehe z. B. K.R. Westerterp, M. Kuczynski, Hydrocarbon Processing, 1986, Nr. 11, Seiten 80 bis 83).

Erfindungsgemäß bevorzugt ist ein Verfahren, bei dem das erzeugte Methanol mittels einer Flüssigkeit aus dem Gleichgewicht extrahiert wird (siehe z. B. K.B. Westerterp, M. Kuczynski, T.M. Bodewes, M.S.A. Vrijland, CHEM.-ING.-TECH. 61, 1989, 3, Seiten 193-199).

Bei einem solchen Verfahren wird das Methanol in mehreren Reaktorstufen erzeugt. Hinter jeder Stufe befindet sich ein Absorber, in dem die Extraktion des Methanols erfolgt. Geeignete Extraktionsmittel sind Polyethylenglykoläther, wobei ein besonders geeignetes Extraktionsmittel Tetraethylenglykoldimethyläther ist. Durch stufenweise Entspannung kann zunächst gelöstes Synthesegas und anschließend das Methanol gasförmig abgetrennt werden. Bei einem 4-stufigen Verfahren entfällt der Synthesegaskreislauf und damit der Kreislaufgas-Kompressor. Etwa 94 % des eingesetzten Kohlenmonoxyds werden im geraden Durchgang umgesetzt. 4-Stufigkeit ist jedoch nicht erforderlich. Erfindungsgemäß kann auch mit 1 bis mehr als 4 Stufen gearbeitet werden, wobei sich jedoch Synthesegasumsetzung und Wirtschaftlichkeit verändern. Die Reaktoren sind im allgemeinen Röhrenreaktoren, es können jedoch auch andere Reaktoren, z. B. sogenannte Abschnittsreaktoren, mit Kaltgaskühlung eingesetzt werden. Kühlwasser und Katalysatorverbrauch sind wesentlich geringer als in einem klassischen Niederdruckverfahren. Das im geraden Durchgang nicht umgesetzte Gas kann dem Vergasungsreaktor, im Falle des Thermoselect-Verfahrens, z. B. dem Vergasungs- oder Homogenisierungsreaktor zugeführt werden.

Hierdurch wird eine anderweitige Entsorgung bzw. Verbrennung des Gases vermieden und durch Umsetzung des rückgeführten Kohlenmonoxids wird zusätzlich Wasserstoff erzeugt.

Die Reinigung des Synthesegases vor Eintritt in die Methanolsynthese kann mittels einer konventionellen Wäsche, wie beispielsweise einer Rectisolwäsche, einer Purisol- oder anderen Wäschen des Standes der Technik erfolgen. Auch die Einstellung des benötigten $CO-H_2$-Verhältnisses kann durch konventionelle Konvertierung, wie z. B. Hochtemperatur- oder Niedertemperaturkonvertierung oder einer Kombination desselben erfolgen.

Da das gemäß vorliegender Erfindung eingesetzte Methanolverfahren auch in Gegenwart relativ großer Mengen Kohlenmonoxid eine praktisch vollständige Umsetzung des Wasserstoffs zu Methanol erlaübt, kann erfindungsgemäß auch ohne Konvertierung gearbeitet werden. Überschüssiges CO am Ausgang der Methanolanlage kann entweder verstromt oder in das Thermoselect-Verfahren rückgeführt werden. Die Fahrweise ohne Konvertierung führt zu erheblichen Kosteneinsparungen.

Erfindungsgemäß kann auch ein Verfahren mit niedrigeren Invest- und Betriebskosten, als sie bei Waschverfahren aufzubringen sind, vorteilhaft eingesetzt werden. Beispielsweise kann eine 2-stufige Oxidation des im Synthesegas enthaltenden Schwefelwasserstoffs zu Schwefel erfolgen, wie sie in der deutschen Patentanmeldung P 4235957 beschrieben ist. Hierbei wird in der ersten Stufe der überwiegende Teil des Schwefelwasserstoffs katalytisch oxidiert. Die Restentschwefelung erfolgt in einer zweiten Stufe mittels mit Jod behandelter Aktivkohle. Das im Synthesegas enthaltene $CO_2$

kann im Anschluß an die Konvertierung durch Druckwechseladsorption selektiv abgetrennt werden. Um in der Druckwechseladsorptionsanlage eine ggf. zeitlich verschobene Desorption von CO und $H_2$ auszugleichen, können die desorbierten Gase vor Eintritt in die Methanolsyntheseanlage entweder in einem Mischtank wieder gemischt werden und ggf. in Zwischentanks zwischengelagert werden oder die Mischung erfolgt dadurch, daß in der ersten Reaktorstufe der Methanolanlage zumindest ein Teil des Synthesegases im Kreislauf gefahren wird.

Auch in diesem Fall der Gasreinigung kann ggf. auf die Konvertierung verzichtet werden, so daß ggf. auch Druckwechseladsorption und Rückmischung nicht erforderlich sind.

Mit Hilfe der Figur soll beispielhaft eine erfindungsgemäße Anordnung näher erläutert werden, wobei, wie für den Fachmann leicht ersichtlich ist, diese Anordnung nicht als limitierend anzusehen ist. Es wird darauf hingewiesen, daß es sich um eine skizzenhafte Darstellung handelt, bei der auf Vollständigkeit verzichtet wird.

1 stellt eine Vergasungsvorrichtung dar, in die über 2 Kohlenstoff enthaltende Materialien zugeführt werden. Über 3 gelangen Sauerstoff ggf. Dampf in Reaktor 1. Bei 4 wird Schlacke abgezogen. In Wärmetauscher 5 wird Dampf erzeugt. 7 sind Quench- bzw. Waschvorrichtungen, in die über 8 Waschwasser zugeführt wird. Bei 9 wird Abwasser abgezogen. Es ist zu beachten, daß die Abkühlung des Synthesesgases auch auf andere Weise erfolgen kann, wie z. B. durch ausschließliches Quenchen oder Dampferzeugung und Heißgasentstaubung. In der Figur ist beispielhaft eine Synthesegasreinigung dargestellt, die aus Schwefelentfernung, Konvertierung und Druchwechseladsorption besteht. Wie bereits oben erläutert, können auch sonstige Reinigungsanlagen eingesetzt werden, wie z. B. konventionelle Waschanlagen.

Aus 7 strömt das Synthesegas in Reaktor 10, in dem Schwefelwasserstoff zu Schwefel oxidiert wird. Bei 11 wird flüssiger Schwefel abgezogen. Eine Nachreinigung zur Entfernung kleiner Restmengen von Schwefelwasserstoff erfolgt in Aktivkohlebett 12. Zu regenerierende Aktivkohle wird bei 13 abgezogen. In Reaktor 14 erfolgt die Konvertierung des Synthesegases zur Einstellung des gewünschten CO-$H_2$-Verhältnisses. Bei 15 wird Wasser zugeführt. Über Wärmetauscher 16 fließt das Synthesegas in den Druckwechseladsorber 17, der im allgemeinen mehrstufig ist. Bei 18 wird $CO_2$ abgezogen. In Mischtank 19 kann, falls erforderlich, Nachmischen zum Erreichen des benötigten CO-$H_2$-Verhältnisses erfolgen. Wie oben bereits erwähnt, kann erfindungsgemäß auch ggf. auf die Konvertierung und ggf. auch auf die Druckwechseladsorption und Rückmischung verzichtet werden. Mittels Kompressor 20 wird das Synthesegas nun in den ersten Methanolreaktor gepumpt. Über Leitung 23 kann im Falle einer Abschnittsreaktors Kaltgas zugeführt werden. Aus Reaktor 22 fließt das Produktgemisch in die erste Extraktionsstufe 24. Der flüssige Methanolextrakt fließt über 25 in Desorptionsstufe 26. Dort wird nach Entspannen bei 27 Methanol dampfförmig abgezogen. Das Extraktionsmittel wird mittels Pumpe 28 und Leitung 29 in die Extraktionsstufen 24, 31 und 33 zurückgepumpt. Das Synthesegas strömt aus 24 bzw. 31 in die Methanolsynthesereaktoren 30 und 32. Aus Extraktionsstufe 33 wird nicht umgesetztes Gas über 34 abgezogen, das wie bereits oben erläutert, in die Vergasungsreaktoren eingeleitet werden kann. Wie bereits oben ausgeführt, kann die Methanolsynthese-Anlage auch aus weniger oder mehr Stufen bestehen, wie z. B. 1, 2, 3, 4 oder mehr Stufen.

## Patentansprüche

1. Verfahren zur Herstellung von Methanol durch Vergasen Kohlenstoff enthaltender Materialien, Reinigung des erzeugten Synthesegases und Umsetzung des Synthesegases zu Methanol, dadurch gekennzeichnet, daß als Kohlenstoff enthaltende Materialien Kohlenstoff enthaltende Abfälle und/oder Reststoffe und/oder aus Reststoffen und Abfällen stammende Sekundärrohstoffe vergast werden und daß die Umsetzung des auf diese Weise erzeugten Synthesegases zu Methanol in einem Reaktorsystem mit Zwischenabzug von Methanol erfolgt.

2. Verfahren nach Anspruch 1, dadurch gekennzeichnet, daß die Zuführung der Kohlenstoff enthaltenden Materialien ohne vorherige Feinzerkleinerung derselben erfolgt.

3. Verfahren nach wenigstens einem der Ansprüche 1 und 2, dadurch gekennzeichnet, daß die Abfallvergasung nach dem Thermoselect-Verfahren erfolgt.

4. Verfahren nach wenigstens einem der Ansprüche 1 und 2, dadurch gekennzeichnet, daß die Abfallvergasung in einem Zyklonvergaser erfolgt.

5. Verfahren nach wenigstens einem der Ansprüche 1 bis 4, dadurch gekennzeichnet, daß die Synthesegasreinigung durch 2-stufige Entschwefelung und ggf. $CO_2$-Entfernung in einer Druckwechseladsorptionsanlage erfolgt.

6. Verfahren nach wenigstens einem der Ansprüche 1 bis 4, dadurch gekennzeichnet, daß die Synthesegasreinigung durch Gaswäsche erfolgt.

**7.** Verfahren nach wenigstens einem der Ansprüche 1 bis 6, dadurch gekennzeichnet, daß das Methanol in einem 4-stufigen Reaktorsystem mit Zwischenabzug erzeugt wird.

**8.** Verfahren nach wenigstens einem der Ansprüche 1 bis 7, dadurch gekennzeichnet, daß der Zwischenabzug des Methanols durch Flüssigextraktion erfolgt.

**9.** Verfahren nach wenigstens einem der Ansprüche 1 bis 8, dadurch gekennzeichnet, daß als Extraktionsmittel Polyethylenglykoläther verwendet wird.

**10.** Verfahren nach wenigstens einem der Ansprüche 1 bis 9, dadurch gekennzeichnet, daß Tetraethylenglykoldimethyläther als Extraktionsmittel verwendet wird.

**11.** Vorrichtung zur Herstellung von Methanol, die eine Thermoselect-Vergasungsanlage für Kohlenstoff enthaltende Abfälle als Einsatzprodukt, eine Synthesegasreinigungsanlage und ein Reaktorsystem mit Zwischenabzug von Methanol enthält.

**12.** Vorrichtung zur Herstellung von Methanol, die einen Zyklonvergaser für Kohlenstoff enthaltende Abfälle als Einsatzprodukt, eine Synthesegasreinigungsanlage und ein Reaktorsystem mit Zwischenabzug von Methanol enthält.

**13.** Vorrichtung nach wenigstens einem der Ansprüche 11 und 12, dadurch gekennzeichnet, daß dieselbe eine Gaswäsche zur Synthesegasreinigung enthält.

**14.** Vorrichtung nach wenigstens einem der Ansprüche 11 bis 13, dadurch gekennzeichnet, daß dieselbe zur Synthesegasreinigung eine 2-stufige Entschwefelungsanlage und eine Druckwechseladsorptionsanlage zur $CO_2$-Entfernung enthält.

**15.** Vorrichtung nach wenigstens einem der Ansprüche 11 bis 14, dadurch gekennzeichnet, daß die Methanolsyntheseanlage ein 4-stufiges Reaktor- und Extraktionssystem enthält.

**16.** Verwendung des Verfahrens nach wenigstens einem der Ansprüche 1 bis 10 zur Erzeugung von Methanol aus Kohlenstoff enthaltenden Abfällen.

Figur

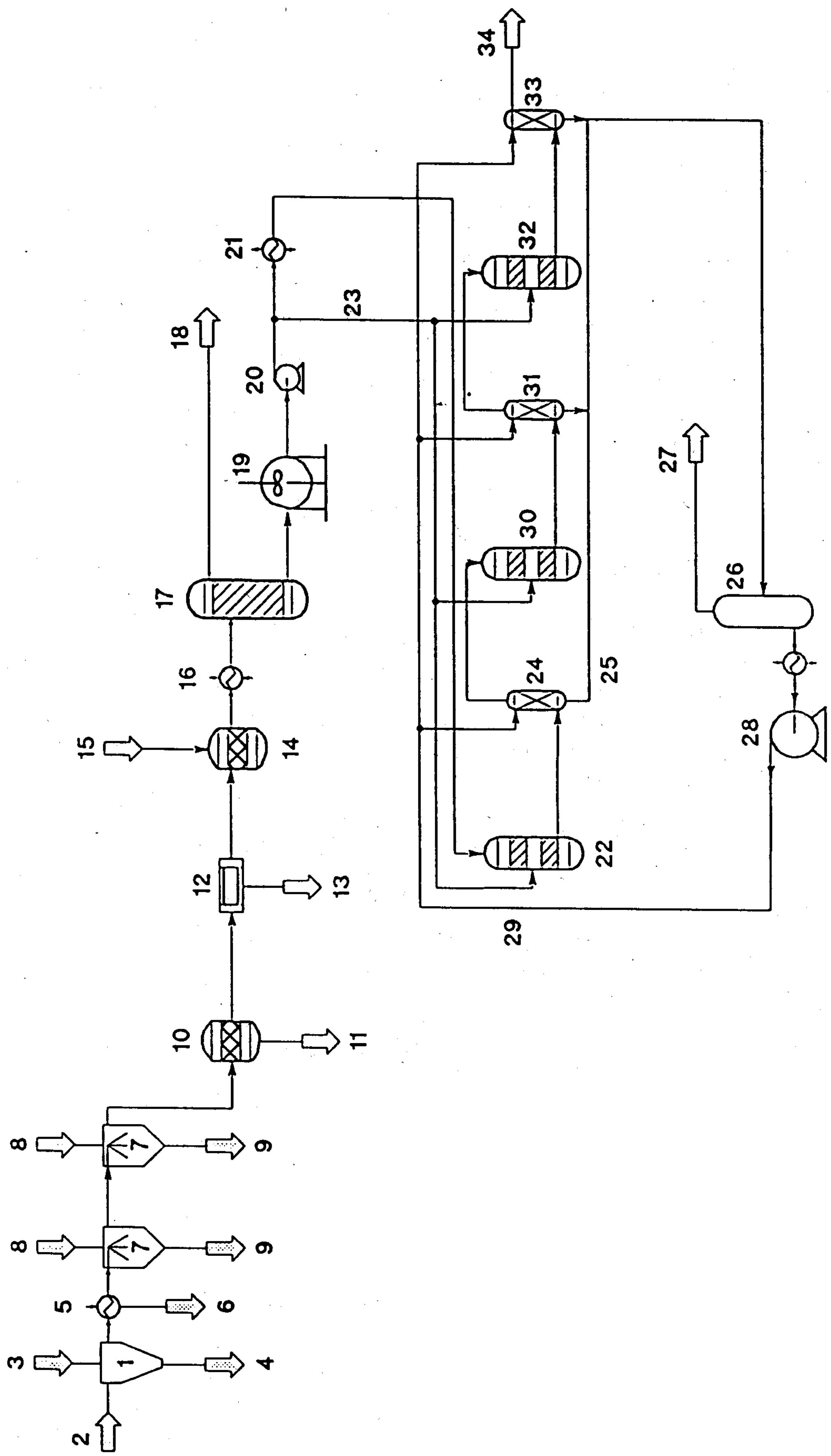

Europäisches Patentamt

# EUROPÄISCHER RECHERCHENBERICHT

Nummer der Anmeldung
EP 93 11 9935

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| Y<br>A | EP-A-0 444 684 (FUNK)<br>* Seite 5, Zeile 30 - Seite 13, Zeile 45 *<br>* Seite 15, Zeile 2-11 *<br>--- | 1<br>2,5,6,16 | C07C29/151 |
| D,Y<br><br><br><br><br>X | CHEM. ING. TECHN.<br>Bd. 61, Nr. 3 , 1989 , WEINHEIM<br>Seiten 193 - 199 XP000053414<br>K.ROEL WESTERTERP 'NEUE KONVERTERSYSTEME FÜR DIE METHANOL-SYNTHESE'<br>* Seite 196, Spalte 2 - Seite 197, Spalte 1 *<br>--- | 1<br><br><br><br><br>7-10 | |
| A | DE-A-28 09 082 (STONE & WEBSTER ENG.)<br>* Seite 1; Anspruch 1 *<br>--- | 1 | |
| A | US-A-5 134 944 (KELLER)<br>* Spalte 10, Zeile 49 - Spalte 12, Zeile 14 *<br>* Spalte 18, Zeile 59 - Spalte 21, Zeile 28 *<br>--- | 1,2,6,16 | |
| | | | RECHERCHIERTE SACHGEBIETE (Int.Cl.5) |
| A | US-A-4 052 173 (SCHULZ)<br>* Spalte 1, Zeile 10-24 *<br>* Spalte 11-12; Anspruch 1 *<br>--- | 1 | C07C<br>C10J |
| A | EP-A-0 411 506 (AIR PRODUCTS AND CHEMICALS)<br>* Seite 17, Zeile 32-50 *<br>--- | 1,5 | |
| D,A | EP-A-0 520 086 (THERMOSELECT)<br>* Seite 9-10; Ansprüche 1-9 *<br>--- | 1-3,6 | |
| D,P,A | EP-A-0 523 447 (OSCHATZ) 20. Januar 1993<br>* Seite 4-5; Ansprüche 1-20 *<br>----- | 1,2,4 | |

Der vorliegende Recherchenbericht wurde für alle Patentansprüche erstellt

| Recherchenort | Abschlußdatum der Recherche | Prüfer |
|---|---|---|
| DEN HAAG | 22. April 1994 | Wendling, J-P |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)